# EUROPEAN PATENT APPLICATION

(11) **EP 1 930 030 A1**
(43) Date of publication of application: **11.06.2008**
(21) Application number: 06810638.4
(22) Date of filing: 27.09.2006
(51) Int. Cl.: A61K 47/32, A61K 9/14, A61K 9/16, A61K 9/20, A61K 9/48, A61K 31/4439, A61K 31/444, A61K 47/38, A61K 47/46

(54) **PULSE PREPARATION HAVING IMPROVED DISINTEGRATION PROPERTIES IN VIVO**

(30) Priority: 29.09.2005 JP 2005283700
(71) Applicant: Eisai R&D Management Co., Ltd., Tokyo 112-8088 (JP)
(72) Inventor: ZAIMA, Yasuhiro, Kawashimatakehaya-machi, Kakamigahara-shi 5016195 (JP); OWAKI, Takayuki, Kawashimatakehaya-machi Kakamigahara-shi 5016195 (JP)
(74) Representative: HOFFMANN EITLE
(86) International application number: PCT/JP2006/319147
(87) International publication number: WO 2007/037259

(57) **Abstract**

An object of the present invention is to provide a pulsed-release preparation that achieves pulsed-release by coating the exterior of a core that contains a physiologically active substance and a disintegrant, with a controlled-release coating that contains a water-insoluble polymer and an enteric polymer, or a water-insoluble polymer and a water-soluble polymer, wherein a satisfactory pulsed-release can be achieved without increasing the amount of disintegrant in the core, in particular even in the low-water environment in the lower part of the digestive tract. The present invention provides a pulsed-release preparation comprising: 1) a core containing a physiologically active substance and a disintegrant; 2) an enteric coating that covers the core; and 3) a controlled-release coating that covers the enteric coating applied on the core and that contains a water-insoluble polymer and an enteric polymer or water-soluble polymer.

## Description

### Technical Field

The present invention relates to a pulsed-release preparation and more particularly relates to a pulsed-release preparation comprising: 1) a core comprising a physiologically active substance and a disintegrant; 2) an enteric coating which covers the core, and which comprises a first enteric polymer; and 3) a controlled-release coating which covers the enteric coating, and which comprises a water-insoluble polymer and a second enteric polymer or a water-soluble polymer.

### Background Art

With the goal of selective drug delivery to an appropriate site within the digestive tract, various investigations have been carried out into oral preparations that exhibit pulsatile release characteristics (pulsed-release preparations) such that, by utilizing the transit time of the preparation within the digestive tract and controlling the active ingredient's release start time, the active ingredient is rapidly released when the preparation has reached a desired site in the digestive tract after a predetermined time period has elapsed post-administration. These preparations can be produced using various formulations and various methods of preparation. In one example of the technology it has been reported that pulsed-release can be achieved by applying, on the surface of a tablet or granule that contains an active ingredient and a disintegrant, a coating that contains a water-insoluble polymer and an enteric polymer, or that contains a water-insoluble polymer and a water-soluble polymer (refer, for example, to Patent Document 1 and Patent Document 2). When it is desired using this type of preparation to achieve pulsed-release of the active ingredient in the lower part of the digestive tract, it is then necessary for the preparation to exhibit a long period of time until a release onset of the active ingredient *in vivo* after ingestion (referred to below as "dissolution lag time"), which in terms of preparation desired has in turn required an increase in the amount of application of the controlled-release coating associated with pulsed-release or an increase in the proportion of the water-insoluble polymer in the controlled-release coating.

However, increasing the amount of application of the controlled-release coating associated with pulsed-release, or increasing the proportion of the water-insoluble polymer in the controlled-release coating, results in an increase in the strength of the controlled-release coating film, which in particular prevents a satisfactory pulsed-release from being achieved in the low-water environment in the lower part of the digestive tract. Another method for pursuing a satisfactory pulsed-release is to increase the swelling power of the tablet by increasing the amount of the disintegrant in the tablet. In this case, however, the tablet weight is increased and the table diameter ends up being large, which impairs the ingestion sensation upon ingestion and lowers compliance.
[Patent Document 1] WO Publication No. WO 03/043661
[Patent Document 2] US Patent Publication No. USP 5,260,069

### Disclosure of Invention

### Problems to be Solved by the Invention

An object of the present invention is to provide a pulsed-release preparation that achieves pulsed-release by coating an exterior of a core that contains a physiologically active substance and a disintegrant, with a controlled-release coating that contains a water-insoluble polymer and an enteric polymer, or a water-insoluble polymer and a water-soluble polymer, wherein a satisfactory pulsed-release can be achieved without increasing the amount of the disintegrant in the core, in particular even in the low-water environment in the lower part of the digestive tract.

When, in particular, an acid-unstable physiologically active substance is used, it is generally necessary to inhibit its release in the stomach and to cause the physiologically active substance to release in the intestines, where the pH is neutral to alkaline. In this case, therefore, the release site for the physiologically active substance is frequently limited to the lower intestinal tract, and therefore there is a need for a preparation that can achieve a satisfactory pulsed-release even in the low-water lower part of the intestinal tract.

### Means for Solving the Problems

In view of these circumstances, the present inventors carried out intensive investigations with respect to a pulsed-release preparation in which a controlled-release coating containing a water-insoluble polymer and an enteric polymer, or a water-insoluble polymer and a water-soluble polymer, is coated on an exterior of a core that contains a physiologically active substance and a disintegrant, in order to search for a pulsed-release preparation that could reliably achieve a satisfactory pulsed-release even after the elapse of a predetermined period of time after ingestion at which the preparation has reached the lower part of the digestive tract. As a result, it was discovered that the initially targeted object could be achieved by the constitution given below, and the present invention was achieved based on this discovery.

That is, in a first aspect of the present invention there is provided a pulsed-release preparation comprising:
1) a core comprising a physiologically active substance and a disintegrant;
2) an enteric coating which covers the core, and which comprises a first enteric polymer; and
3) a controlled-release coating which covers the enteric coating, and which comprises a water-insoluble polymer and a second enteric polymer or a water-soluble polymer.

In a second aspect of the present invention there is provided a process for producing a pulsed-release preparation, comprising the steps of:
forming an enteric coating which covers a core, and which comprises a first enteric polymer, the core comprising a physiologically active substance and a disintegrant; and
forming a controiled-release coating which covers the enteric coating, and which comprises a water-insoluble polymer and a second enteric polymer or a water-soluble polymer.

### Advantageous Effects of the Invention

With regard to pulsed-release preparation that achieves pulsed-release of a a physiologically active substance by coating the exterior surface of the core that contains the physiologically active substance and a disintegrant with a controlled-release coating that contains a water-insoluble polymer and an enteric polymer, or a water-insoluble polymer and a water-soluble polymer, and in particular with regard to the use of acid-unstable physiologically active substances, the present invention, by providing an enteric polymer-containing enteric coating between the core and the aforementioned controlled-release coating can produce a pulsed-release preparation that can achieve a satisfactory pulsed-release even under the low-water environment in the lower part of the digestive tract.

### Best Mode for Carrying Out the Invention

The following embodiments are examples for the purpose of explaining the present invention, but the present invention should not be construed as limited only to these embodiments. The present invention can be implemented using various modifications without departing from the gist of the present invention.

A first aspect of the present invention provides a pulsed-release preparation comprising: 1) a core comprising a physiologically active substance and a disintegrant; 2) an enteric coating which comprises a first enteric polymer, and which covers the core; and 3) a controlled-release coating which covers the enteric coating, and which comprises a water-insoluble polymer and a second enteric polymer or a water-soluble polymer.

The term "core" used in the present invention refers to a central substance that contains a physiologically active substance alone or in combination with one or more preparation excipients and generally takes the form of a tablet, granule, fine granule, and the like.

The disintegrant used in the present invention that is contained in the core has the ability to absorb water and thereby undergo an expansion in volume, but is not otherwise particularly limited. One or more disintegrants may be contained in the core. Examples of the disintegrant used in the present invention include, but are not limited to, crosspovidone, low-substituted hydroxypropyl cellulose, crosscarmellose sodium, carmellose calcium, and the like, preferably crosspovidone or low-substituted hydroxypropyl cellulose. Crosspovidone is more preferred in the particular case of benzimidazole-type compounds, not only just for its swelling characteristics as a disintegrant, but also because it has a substantial stabilizing activity in that it can inhibit degradation-induced discoloration of benzimidazole-type compounds. A content of the disintegrant is generally from 1 to 50 % by weight, preferably from 5 to 40 % by weight, and more preferably from 10 to 35 % by weight, based on the weight of the core. More particularly, a content of crosspovidone content for the benzimidazole-type compound is preferably from 10 to 1000 % by weight, more preferably from 20 to 800 % by weight, even more preferably from 50 to 500 % by weight, and particularly preferably from 100 to 300 % by weight, based on the weight of the benzimidazole-type compound

The core further comprises various preparation excipients. Examples of the excipients optionally include the generally known diluents, binders, lubricants, and the like.

The "core" used in the present invention can be produced by the commonly used methods. For example, crosspovidone and sodium hydroxide as a stabilizer can be mixed into the benzimidazole-type compound; diluent and binder are added thereto; and dry granulation or wet granulation, such as high shear granulation, extrusion granulation, and the like, is carried out. In addition, production can as necessary be carried out by compression with the addition of a disintegrant and a lubricant. Of course, these examples should not be construed as a limitation to these methods.

The term "enteric coating" used in the present invention refers to a coating that does not dissolve in acidic solutions and that does dissolve at neutral to basic pH values, but is not otherwise particularly limited. It generally has an enteric polymer as its main component. The enteric polymer is not particularly limited and can be exemplified by one or more polymers selected from the group consisting of hydroxypropyl methylcellulose phthalate, hydroxypropyl methylcellulose acetate succinate, methacrylic acid-methyl methacrylate copolymers (Eudragit L (Rohm Pharma), Eudragit S (Rohm Pharma)), and methacrylic acid-ethyl acrylate copolymers (Eudragit LD (Rohm Pharma)); methacrylic acid-methyl methacrylate copolymers (Eudragit L, Eudragit S) or hydroxypropyl methylcellulose phthalate being preferred.

The term "controlled-release coating" used in the present invention refers to a "controlled-release coating that covers the enteric coating applied on the core and that has as its main component a water-insoluble polymer and an enteric polymer, or a water-insoluble polymer and a water-soluble polymer". Through the application, on the enteric coating applied on the core, of the controlled-release coating whose main component is the water-insoluble polymer and the enteric polymer, or the water-insoluble polymer and the water-soluble polymer, the physiologically active substance-containing pulsed-release preparation, and particularly an acid-unstable pulsed-release preparation can be produced, that exhibits highly reliable dissolution characteristics and that has a prolonged dissolution lag time.

The water-insoluble polymer in the "controlled-release coating" used in the present invention is almost completely insoluble in water and undergoes dissolution or uniform dispersion in organic solvent such as methanol, ethanol, propanol, isopropanol, acetone, and the like, but is not otherwise particularly limited. Preferred examples of the water-insoluble polymer include ethyl cellulose, aminoalkyl methacrylate copolymer RS (Eudragit RS (Rohm Pharma)), and/or shellac and the like, ethyl cellulose being more preferred. A single one of these can be used by the present invention or two or more can be used in combination.

The enteric polymer in the "controlled-release coating" used in the present invention is not particularly limited and can be exemplified by one or more polymers selected from the group consisting of hydroxypropyl methylcellulose phthalate, hydroxypropyl methylcellulose acetate succinate, methacrylic acid-methyl methacrylate copolymers (Eudragit L (Rohm Pharma), Eudragit S (Rohm Pharma)), and methacrylic acid-ethyl acrylate copolymers (Eudragit LD (Rohm Pharma)); methacrylic acid-methyl methacrylate copolymers (Eudragit L, Eudragit S) and methacrylic acid-ethyl acrylate copolymers (Eudragit LD) being preferred and methacrylic acid-methyl methacrylate copolymers (Eudragit L) being more preferred.

The water-soluble polymer in the "controlled-release coating" used in the present invention is not particularly limited and can be exemplified by hydroxypropyl cellulose, methyl cellulose, sodium carboxymethyl cellulose, hydroxypropyl methylcellulose, and polyvinylpyrrolidone, at least one selected from this group being preferred.

The dissolution lag time due to the "controlled-release coating" can be adjusted using the composition of the controlled-release coating (proportion of the water-insoluble polymer, enteric polymer, or water-soluble polymer) and the film thickness of the coating. In addition, by coating the enteric polymer-containing enteric coating to the inside of the controlled-release coating, the pulsed-release preparation having a prolonged lag time can be reliably produced by the present invention and reliable release even in the lower part of the digestive tract can be achieved.

The total content of the water-insoluble polymer and enteric polymer in the controlled-release coating is not particularly limited and, is generally from 30 to 85 % by weight, preferably from 40 to 75 % by weight, and more preferably from 50 to 65 % by weight, based on the weight of the controlled-release coating. The content of the water-insoluble polymer in the "controlled-release coating" containing water-insoluble polymer and enteric polymer is also not particularly limited and, is generally from 3.0 to 95 % by weight, preferably from 5.0 to 90 % by weight, and more preferably from 10 to 85 % by weight, based on the total content of the water-insoluble polymer and enteric polymer in the controlled-release coating. The content of the water-insoluble polymer in the "controlled-release coating" containing water-insoluble polymer and water-soluble polymer is also not particularly limited and, is generally from 3.0 to 95 % by weight, preferably from 5.0 to 90 % by weight, and more preferably from 10 to 85 % by weight, based on the total content of the water-insoluble polymer and enteric polymer in the controlled-release coating.

A preferred aspect of the "controlled-release coating" in the present invention contains ethyl cellulose as the water-insoluble polymer and methacrylic acid-methyl methacrylate copolymer (Eudragit L, Eudragit S) as the enteric polymer. Another preferred aspect of the "controlled-release coating" in the present invention contains ethyl cellulose as the water-insoluble polymer and hydroxypropyl cellulose as the water-soluble polymer.

The "controlled-release coating" according to the present invention preferably contains a fat-soluble wax and/or a plasticizer. The fat-soluble wax used in the present invention is not particularly limited and is exemplified by magnesium stearate, calcium stearate, stearic acid, carnauba wax, glyceryl dibehenate, and sucrose fatty acid esters that have an HLB value no greater than 5, and also by waxes such as glyceryl fatty acid esters, white beeswax, hydrogenated oil, microcrystalline wax, and the like. It is preferably at least one selected from the group consisting of magnesium stearate, calcium stearate, stearic acid, carnauba wax, glyceryl dibehenate, and hydrogenated oil and more preferably magnesium stearate or calcium stearate.

The plasticizer used in the present invention is not particularly limited and can be exemplified by triethyl citrate, cetyl alcohol, glyceryl fatty acid esters, propylene glycol, and the like; a single one of these may be used or two or more may be used in combination. Cetyl alcohol or triethyl citrate is preferred. When there is a large proportion of the water-insoluble polymer in the total content of water-insoluble polymer and enteric polymer, cetyl alcohol is preferably blended as the plasticizer; when, on the other hand, there is a small proportion of the water-insoluble polymer, triethyl citrate is preferably blended as the plasticizer. The content of the plasticizer in the controlled-release coating is not particularly limited and, is generally from 0.1 to 20 % by weight, preferably from 0.5 to 15 % by weight, and more preferably from 1.0 to 15 % by weight, based on the weight of the controlled-release coating.

The present invention, through its application of the enteric coating to the inside of the controlled-release coating which controls the pulsed-release, provides a pulsed-release preparation that has a prolonged dissolution lag time. This preparation possesses the excellent characteristic of being able to provide an extension of the dissolution lag time without increasing the amount of application of the controlled-release coating and without increasing the content of the water-insoluble polymer in the controlled-release coating. In addition, since the enteric polymer dissolves *in vivo* in the intestinal tract where the pH is from neutral to alkaline, the coating strength at the time of pulsed-release is reduced. Accordingly, a reliable release of the physiologically active substance can be induced and an excellent *in vivo* disintegrability can be obtained even for the pulsed-release preparation that has a prolonged dissolution lag time.

The present invention also provides a pulsed-release preparation that additionally contains an inactive intermediate coating between the enteric coating and the controlled-release coating. The present invention further provides a pulsed-release preparation that additionally contains an inactive intermediate coating between the core and the enteric coating.

The term "inactive intermediate coating" used in the present invention, when present between the enteric coating that covers the core and the controlled-release coating which is coated on the enteric coating coated on the core and which comprises the water-soluble polymer and the enteric polymer or the water-soluble polymer, refers to a coating that prevents interaction between the two coatings and that does not exercise a negative influence on the stability of either of the two coatings. When present, on the other hand, between the core and the enteric coating, the term "inactive intermediate coating" used in the present invention refers to a coating that does not exercise a negative influence on the stability of the physiologically active substance present in the core.

The term "dissolution lag time" used in the present invention refers, in an in *vitro* test, to the amount of time that elapses after the start of the dissolution test until dissolution of the physiologically active substance from the preparation in the test solution begins, while in an *in vivo* test it refers to the time until release of the physiologically active substance after ingestion of the preparation.

The term "physiologically active substance" used in the present invention is not particularly limited; however, the pulsed-release preparation according to the present invention is particularly useful when the physiologically active substance is an acid-unstable physiologically active substance. The term "acid-unstable physiologically active substance" refers to a physiologically active substance that is chemically unstable and easily degrades at acidic pH and/or the acidic pH in the stomach.

Specific examples of the "acid-unstable physiologically active substance" used in the present invention include, but are not particularly limited to, peptic ulcer therapeutics, antibiotics, analgesics, anti-dementia drugs, anti-platelet drugs, antidepressants, agents that improve cerebral blood flow and metabolism, anti-allergics, and the like. Examples of known peptic ulcer therapeutics are benzimidazole-type compounds that exercise an inhibitory action on the proton pump and strongly inhibit gastric acid secretion, as well as the pharmacologically acceptable salts of such benzimidazole-type compounds, and specific examples thereof are rabeprazole (I), omeprazole (II), emesoprazole (III), lansoprazole (IV), pantoprazole (V), and tenatoprazole (VI), as represented by the chemical structures shown below, as well as their alkali metal salts and alkaline-earth metal salts. The sodium salt and potassium salt are preferred for the alkali metal salt, while the magnesium salt is preferred for the alkaline-earth metal salt. Rabeprazole sodium is particularly preferred as the peptic ulcer therapeutic.

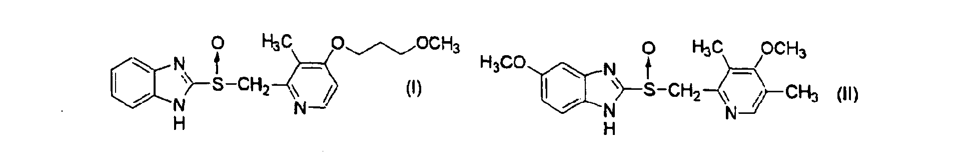

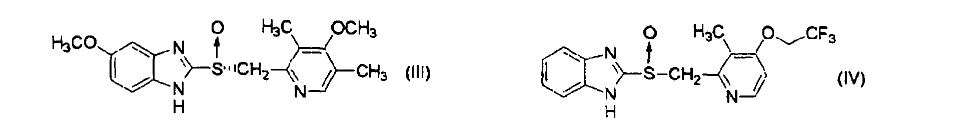

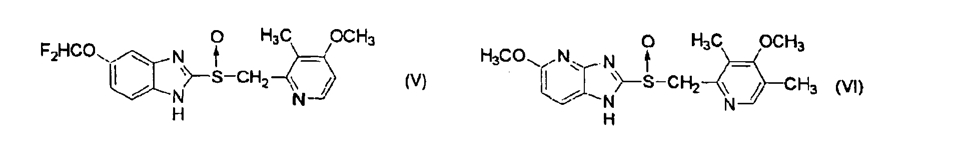

The pulsed-release preparation according to the present invention preferably comprises at least one basic additive in its core as a stabilizer for the "acid-unstable physiologically active substance". For example, the aforementioned benzimidazole-type compounds are very unstable under acidic conditions, and degradation products are readily produced and the preparation is prone to discoloration when the preparation containing such a compound is exposed to warm, moist conditions. Moreover, while the benzimidazole-type compounds are unstable in the acidic pH range, their stability in the neutral pH range varies with the particular drug. For example, the half-life at pH 7 is 23 hours for omeprazole, 13 hours for lansoprazole, 39 hours for pantoprazole, and 30 minutes for rabeprazole. As a consequence, the potential exists for rabeprazole and the like to undergo degradation when intestinal fluid infiltrates into the core. Therefore, the stability of an acid-unstable physiologically active substance can be securely maintained by blending a basic additive, e.g., sodium hydroxide, in the core and thereby maintaining the interior of the core basic even when intestinal fluid has infiltrated into the core. Specific examples of the basic additive include, but are not limited to, sodium hydroxide, potassium hydroxide, magnesium oxide, calcium oxide, magnesium hydroxide, calcium hydroxide, sodium carbonate, sodium phosphate, potassium carbonate, and the like; sodium hydroxide, potassium hydroxide, magnesium oxide, calcium oxide, magnesium hydroxide, and calcium hydroxide being preferred and sodium hydroxide and magnesium oxide being more preferred.

The blending ratio between the benzimidazole-type compound and sodium hydroxide or potassium hydroxide serving as the basic additive, is generally from 0.1 to 40 % by weight, preferably from 1.0 to 20 % by weight, and more preferably from 2.0 to 15 % by weight, based on the weight of the benzimidazole-type compound. For the use of a basic additive other than sodium hydroxide or potassium hydroxide, the blending ratio is generally from 10 to 5000 % by weight, preferably from 100 to 2000 % by weight, and more preferably from 200 to 1000 % by weight, based on the weight of the benzimidazole-type compound.

With regard to the application of the enteric coating, controlled-release coating, or inactive intermediate coating which are used in the present invention, the solvent in the corresponding coating solution should have the ability to dissolve or uniformly disperse the enteric polymer, water-insoluble polymer, water-soluble polymer, and the like, but is not otherwise particularly limited. Examples of the solvent include water, methanol, ethanol, propanol, isopropanol, acetone, and the like; methanol, ethanol, propanol, and isopropanol being preferred, and ethanol and isopropanol being more preferred. One or more of these solvents may be used, and suitable mixtures may be employed.

The enteric coating used in the present invention contains an enteric polymer as its main compound, and because it therefore exhibits acidity, its direct contact with a benzimidazole-type compound, which is an acid-unstable physiologically active substance, is undesirable. It is therefore preferred for the pulsed-release preparation according to the present invention that an inactive intermediate coating that does not negatively influence the stability of the benzimidazole-type compound be provided between the enteric coating and the core containing the benzimidazole-type compound. The inactive intermediate coating may also be disposed between the enteric coating and the controlled-release coating. The inactive intermediate coating is not particularly limited and is generally a coating that contains a water-soluble polymer, a water-insoluble polymer, and/or a water-dispersible substance. Specific examples of the inactive intermediate coating used in the present invention include, but are not limited to, hydroxypropyl cellulose, hydroxypropyl methylcellulose, aminoalkyl methacrylate copolymers, ethyl cellulose, lactose, mannitol, crystalline cellulose, and the like. Moreover, an intermediate coating comprising water-insoluble microparticles dispersed in a water-insoluble polymer, as disclosed in Japanese Patent Application Laid-open No. Hei 01-029062, may be applied.

From the perspective of the release property and bioavailability of the active ingredient present in the preparation and the moisture resistance of the preparation itself, the pulsed-release preparation according to the present invention, particularly the pulsed-release preparation containing rabeprazole sodium as an acid-unstable physiologically active substance, preferably comprises controlled-release coating that contains Eudragit L or S and ethyl cellulose wherein the ethyl cellulose blending proportion is from 10 to 25 % by weight and preferably from 11 to 20 % by weight, based on the total content of the Eudragit L or S and ethyl cellulose present in the controlled-release coating, and that contains calcium stearate at from 10 to 35 % by weight and preferably from 20 to 35 % by weight based on the weight of the controlled-release coating and triethyl citrate at from 6.0 to 15 % by weight and preferably from 7.5 to 12 % by weight based on the weight of the controlled-release coating.

The dosage form of the pulsed-release preparation according to the present invention can be, for example, a tablet, granule, or fine granule, but is not particularly limited as long as it is a solid preparation.

A solid oral preparation of an acid-unstable physiologically active substance can be implemented as a capsule formulation by filling a capsule with a pulsed-release preparation according to the present invention and an enteric preparation comprising an enteric coating applied on a core that contains the aforementioned acid-unstable physiologically active substance. From the perspective of the efficacy in the patient whose has ingested the preparation, this makes it possible to provide both a rapid effect due to the enteric preparation and persistence due to the pulsed-release preparation. That is, a preparation can be provided that has both a rapid effect due to the enteric preparation and delayed effect after a predetermined time lag due to the pulsed-release preparation. The capsule used in the present invention may be a hard capsule or a soft capsule; the capsule material is also not particularly limited and can be exemplified by gelatin, hydroxypropyl methylcellulose, pullulan, and the like. The capsule may be filled, in numerical terms, with a single pulsed-release preparation or a plurality of pulsed-release preparations and with a single enteric preparation or a plurality of enteric preparations. For example, a hard capsule may be filled with a plurality of size-reduced minitablets of the enteric preparation and a plurality of size-reduced minitablets of the pulsed-release preparation, or may be filled with granules or fine granules of the pulsed-release preparation and the enteric preparation, or may be filled with the pulsed-release preparation in tablet form and granules or fine granules of the enteric preparation, or may be filled with granules or fine granules of the pulsed-release preparation and the enteric preparation in tablet form.

The present invention also provides a method of producing a pulsed-release preparation, comprising the steps of: forming an enteric coating that covers a core containing a physiologically active substance and a disintegrant; and forming a controlled-release coating which covers enteric coating applied on the core and that contains a water-insoluble polymer and an enteric polymer or water-soluble polymer. The pulsed-release preparation according to the present invention can be produced, for example, by the following method. Physiologically active substance, diluent, and disintegrant (internal addition) are charged into a high shear granulator and mixed until the individual components are dispersed uniformly. Then, while the materials in the high shear granulator are being mixed, ethanol containing a basic additive dissolved therein is added and wet granulation is carried out. The granulated materials are then dried in a tray drier and after drying the granules are milled to adjust the size by a milling device. The milled granules, disintegrant (external addition), and lubricant are charged into a rotating vessel-type blender and blended until the individual components are dispersed uniformly. The resulting mixture is then compressed by a rotary tablet press to yield tablets that function as the core.

A coating solution, prepared by dissolving a water-soluble polymer in ethanol, is sprayed onto the core using a pan coater in order to additionally provide an inactive intermediate coating on the core. A coating solution prepared by dissolving an enteric polymer and a plasticizer in ethanol is additionally sprayed onto the core using a pan coater in order to provide an enteric coating on the intermediate coating that covers the core. A coating fluid prepared by dissolving an insoluble polymer, enteric polymer or water-soluble polymer, and plasticizer in ethanol and dispersing a fat-soluble wax in the ethanol, is then sprayed onto the core using a pan coater to provide a controlled-release coating on the enteric coating that covers the core and thereby yield the pulsed-release preparation.

Test examples in accordance with the present invention are described below in order to illustrate the effects produced by the present invention.

### 1. Effect in vitro of the enteric coating on the pulsed-release of physiologically active substance

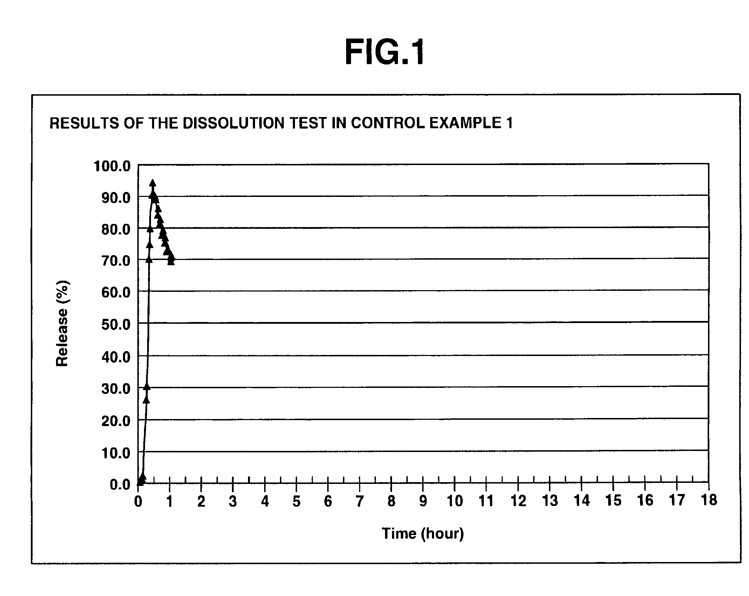

A tablet composition (see Table 1) containing rabeprazole sodium as the physiologically active substance and crosspovidone as a disintegrant was coated with an enteric coating and additionally with a controlled-release coating containing ethyl cellulose (water-insoluble polymer) and Eudragit L-100 (enteric polymer) (see Tables 2 and 3). Dissolution tests were carried out in Examples 1 to 3, which were pulsed-release preparations according to the present invention. The dissolution test using the preparations according to the examples was carried out in accordance with the dissolution test method of the Japanese Pharmacopoeia IV at 50 rpm by the paddle method in pH 6.8 buffer after the tablet had been soaked for 2 hours in pH 1.2 buffer; the timewise change in the percentage dissolved of the rabeprazole sodium from the preparation was evaluated. The preparations of Control Example 1 and Control Example 2 were subjected to the same evaluation to provide comparative examples. The results are shown in Figures 1 to 3.

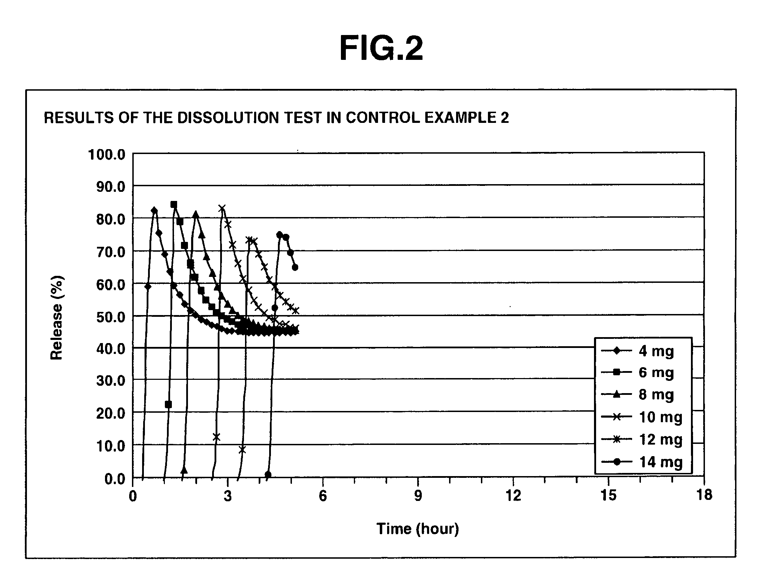

As is clear from the results shown in Figure 1, release of the physiologically active substance was finished within 1 hour for the preparation of Control Example 1. This was a preparation in which 1) a core containing physiologically active substance and a disintegrant was coated with 2) an enteric coating and an inactive intermediate layer was additionally present between the enteric coating and the core, but which lacked 3) a controlled-release coating containing a water-insoluble polymer and an enteric polymer or water-soluble polymer. In addition, as is clear from the results shown in Figure 2, for the preparation of Control Example 2, which was a preparation in which 1) a core containing physiologically active substance active substance and a disintegrant was coated with 3) a controlled-release coating containing a water-insoluble polymer and an enteric polymer or water-soluble polymer with an inactive intermediate layer provided between the core and the controlled-release coating, a larger coating amount for the controlled-release coating resulted in an increase in the dissolution lag time in the dissolution test, but the dissolution lag time was less than 6 hours even when a 14 mg coating had been provided.

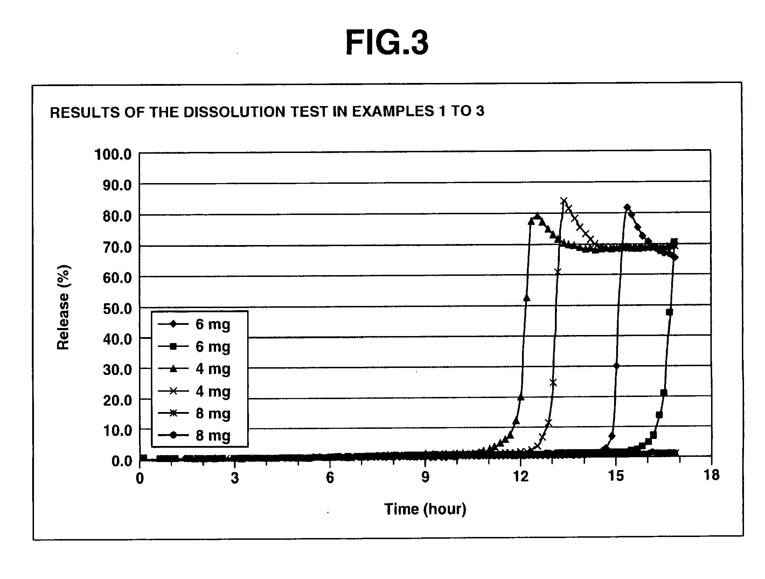

In contrast, in the case of a preparation according to the present invention (Examples 1 to 3), that is, the pulsed-release preparation that comprises 1) a core containing a physiologically active substance and a disintegrant, 2) an enteric coating that covers the core, and 3) a controlled-release coating that covers the enteric coating applied on the core and that contains a water-insoluble polymer and an enteric polymer or water-soluble polymer, and that additionally comprises an inactive intermediate coating provided between the core and the controlled-release coating, the results shown in Figure 3 demonstrate that the dissolution lag time of at least 12 hours was obtained when the coated amounts of the controlled-release coating were 4, 6, or 8 mg (total coated amount including 8 mg for the enteric coating = 12, 14, or 16 mg). As shown in Figure 3, release of the physiologically active substance was not observed within the observation period of this experiment for the preparation in which the coated amount of the controlled-release coating was 8 mg.

It was demonstrated for the preparations according to the present invention, in which the enteric coating is applied to the inside of the controlled-release coating which controls the pulsed-release, that the enteric coating causes a substantial increase in the dissolution lag time in the pulsed-release of the physiologically active substance.
Table 1.

**Table 1 The core composition (unit: mg)**

| | | |
|---|---|---|
| **rabeprazole sodium** | **active ingredient** | **10.0** |
| **D-mannitol** | **diluent** | **24.6** |
| **NaOH** | **stabilizer** | **0.5** |
| **crosspovidone XL (internal addition)** | **disintegrant** | **15.0** |
| **crosspovidone XL (external addition)** | **disintegrant** | **1.5** |
| **sodium stearyl fumarate** | **lubricant** | **0.9** |
| **total** | | **52.5** |

Table 2.

**Table 2**

| **Coating fluid compositions for Examples 1 to 3 and Control Examples 1 and 2** | | |
|---|---|---|
| **type of coating** | **component name** | **%** |
| **inactive intermediate coating** | **hydroxypropyl cellulose** | **75** |
| | **magnesium stearate** | **25** |
| | **ethanol** | **q. s.** |
| | **hydroxypropyl methylcellulose phthalate** | **80** |
| **enteric coating** | **pigment blend** | **12** |
| | **glyceryl fatty acid ester** | **8** |
| | **80% ethanol** | **q. s.** |
| | **Eudragit L** | **8** |
| | **ethyl cellulose** | **45** |
| **Controlled-release coating** | **talc** | **8** |
| | **titanium oxide** | **5** |
| | **cetyl alcohol** | **3** |
| | **magnesium stearate** | **31** |
| | **ethanol** | **q. s.** |

Table 3.

**Table 3**

| **Coating amounts (mg/tablet) in the individual experiments** | | | |
|---|---|---|---|
| **experiment** | **intermediate coating** | **enteric coating** | **coating** |
| **Control Example 1** | **3** | **8** | **-** |
| **Control Example 2** | **3** | **-** | **4, 6, 8, 10, 12, 14** |
| **Example 1** | **3** | **8** | **4** |
| **Example 2** | **3** | **8** | **6** |
| **Example 3** | **3** | **8** | **8** |

### 2. Effect of an inactive intermediate coating between the enteric coating and the controlled-release coating on the pulsed-release of physiologically active substance

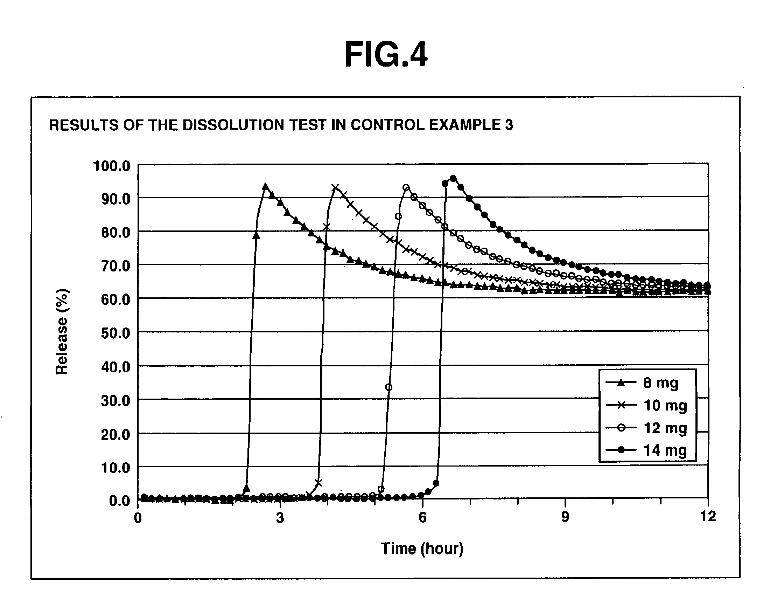

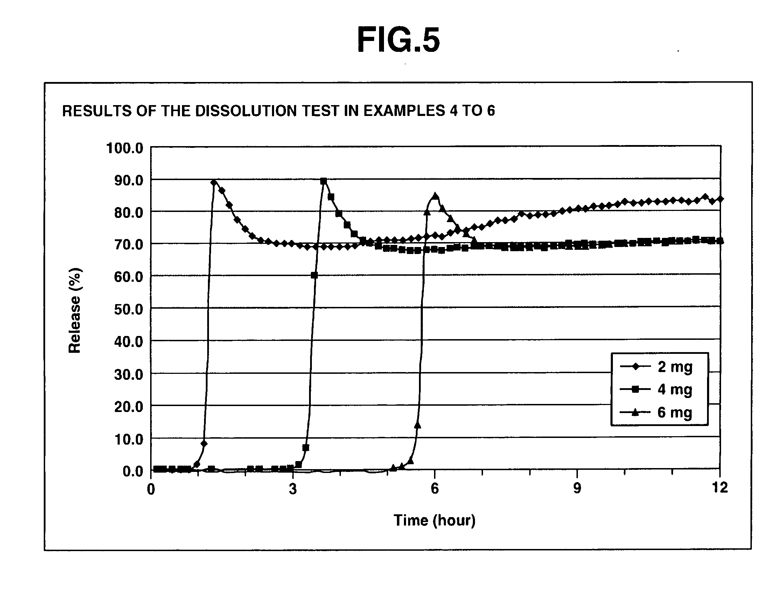

Dissolution testing was carried out in Examples 4 to 6, which were pulsed-release preparations according to the present invention prepared by coating a tablet composition (see Table 1) with an enteric coating and then an inactive intermediate coating and thereafter with a controlled-release coating containing ethyl cellulose (water-insoluble polymer) and Eudragit L-100 (enteric polymer) (see Tables 4 and 5). The dissolution test using the preparations according to the examples was carried out at 50 rpm by the paddle method in pH 6.5 buffer after the tablet had been immersed for 2 hours in pH 1.2 buffer; the timewise change in the percentage dissolved of the rabeprazole sodium from the preparation was evaluated. The preparation of Control Example 3 was subjected to the same evaluation to provide a comparative example. The results are shown in Figures 4 and 5.

As is clear from the results shown in Figure 4, with the preparation of Control Example 3, which was a preparation in which 1) a core containing physiologically active substance and a disintegrant was coated with 3) a controlled-release coating containing a water-insoluble polymer and an enteric polymer or water-soluble polymer with an inactive intermediate layer additionally provided between the core and the controlled-release coating, but which lacked the enteric coating that covered the core, the dissolution lag time did not exceed 7 hours even when a 14 mg controlled-release coating was provided.

In contrast, in the case of the preparations according to Examples 4 to 6 of the present invention, that is, a pulsed-release preparation that comprises 1) a core containing a physiologically active substance and a disintegrant, 2) an enteric coating that covers the core, and 3) a controlled-release coating that covers the enteric coating applied on the core and that contains a water-insoluble polymer and an enteric polymer or water-soluble polymer, and that additionally comprises an inactive intermediate coating provided between the enteric coating and the controlled-release coating and also an inactive intermediate coating provide between the core and the enteric coating, the results shown in Figure 5 demonstrate, for example, that the dissolution lag time of at least 5 hours was obtained even for the application of a 6 mg controlled-release coating in Example 6, and thus that a prolonged dissolution lag time was obtained by the application of small amounts of the controlled-release coating.

Just as for the preparation in which the controlled-release coating and the enteric coating are provided, an increase in the dissolution lag time was also confirmed for the preparation in which an intermediate coating of an inactive water-soluble polymer was provided between the controlled-release coating and the enteric coating.
Table 4.

**Table 4**

| **Coating fluid compositions for Examples 4 to 6 and Control Example 3** | | |
|---|---|---|
| **type of coating** | **component name** | **%** |
| **inactive intermediate coating 1 (between the enteric coating)** | **hydroxypropyl cellulose** | **75** |
| | **calcium stearate** | **25** |
| | **ethanol** | **q. s.** |
| | **hydroxypropyl methylcellulose phthalate** | **80** |
| **enteric coating** | **pigment blend** | **12** |
| | **glyceryl fatty acid ester** | **8** |
| | **80% ethanol** | **q. s.** |
| **inactive intermediate coating 2 (between the enteric and controlled-release coatings)** | **hydroxypropyl methylcellulose** | **80** |
| | **talc** | **20** |
| | **60% ethanol** | **q. s.** |
| | **Eudragit L** | **45** |
| | **ethyl cellulose** | **8** |
| | **talc** | **8** |
| **Controlled-release coating** | **titanium oxide** | **5** |
| | **trietyl citrate** | **3** |
| | **calcium stearate** | **31** |
| | **ethanol** | **q. s.** |

Table 5.

**Table 5**

| **Coating amounts (mg) and lag times in pH 6.8 buffer in the individual experiments** | | | | |
|---|---|---|---|---|
| **experiment** | **inactive intermediate coating 1** | **enteric coating** | **inactive intermediate 2** | **Controlled-release coating** |
| **Control 3 Example 3** | | **-** | **-** | **8, 10, 12, 14** |
| **Example 4** | **3** | **3** | **2** | **2** |
| **Example 5** | **3** | **3** | **2** | **4** |
| **Example 6** | **3** | **3** | **2** | **6** |

### 3. Effect in vivo of the enteric coating on pulsed-release of the physiologically active substance

The pulsed-release preparation of Example 7 (dissolution lag time = 10 hours) was administered to beagles; the fecal output over 24 hours was collected; and the rupture status of the controlled-release coating and the enteric coating was checked for the tablets in the feces (observation of the excreted material in the feces at the 5th, 8th, and 24th hour after administration of the pulsed-release preparation). The beagles were fasted for at least 12 hours and pentagastrin was administered (0.1 mL/animal, s.c.) 30 minutes before administration of the pulsed-release preparation. In the control example, the same evaluation was carried out using Control Example 4 (dissolution lag time = 3 hours), which lacked the enteric coating. The preparations are shown in Table 6.

The results are shown in Table 7. Numerous fragments of the coating film were observed with Control Example 4, which lacked the enteric coating. In contrast, with the pulsed-release preparation of Example 7, in which in accordance with the present invention the enteric coating was applied to the inside of the controlled-release coating, traces of the coating were not observed, notwithstanding the fact that its dissolution lag time was as much as more than 3 times longer, and complete disintegration or dissolution *in vivo* was thus confirmed.

The pulsed-release preparation according to the present invention, through the application of the enteric coating to the inside of the controlled-release coating which controls the pulsed-release, provides the pulsed-release preparation that has a prolonged dissolution lag time, and in relation thereto clearly exhibits excellent *in vivo* disintegration characteristics that reliably bring about release of physiologically active substance in the lower part of the digestive tract.
Table 6.

**Table 6**

| **Coating amounts (mg) and lag times in pH 6.8 buffer in the individual experiments** | | | | |
|---|---|---|---|---|
| **experiment** | **intermediate coating** | **enteric coating** | **Controlled-release coating** | **lag time (hr)** |
| **Control Example 4** | **3** | **-** | **12** | **3** |
| **Example 7** | **3** | **8** | **4** | **10** |

Table 7.

**Table 7**

| **Results of the observation of coating status in beagle faces** | | |
|---|---|---|
| **experiment** | **N** | **coating status** |
| | **1** | **numerous coating fragments** |
| **Control Example 4** | **2** | **numerous coating fragments** |
| | **3** | **numerous coating fragments** |
| | **1** | **no traces of the coating** |
| **Example 7** | **2** | **no traces of the coating** |
| | **3** | **no traces of the coating** |

### Examples

Examples and control examples are provided below in order to describe the present invention in detail; however, the present invention should not be construed as being limited thereto.

The excipients described in the following examples either conformed to the standards in the Japanese Pharmacopoeia, Japanese Pharmaceutical Excipients 2003, the Japanese Pharmaceutical Codex 1997, and the like, or were reagents.

### Example 1

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of granules using a milling device (Power Mill, Dalton Co., Ltd.) and a 1 mmφ mesh screen. The milled granules, 300 g of crosspovidone, and 180 g of sodium stearyl fumarate were then mixed for 50 minutes at 20 rpm using a rotating vessel-type blender (50 L Tumbler Mixer, Tokuju Seisakusho). The blended granules were compressed (4.8 mmφ, 4.8 mmR) at a compression force of 500 kgf using a rotary tablet press (AP-15, Hata Tekko) to obtain a core.

3000 g of the core was charged into a pan coater (Aqua Coater Model 48, Freund Corporation) and was sprayed at an inlet air temperature of 60°C with a coating fluid prepared by dissolving 186 g of hydroxypropyl cellulose and dispersing 62 g of magnesium stearate in 3600 g of ethanol, to apply a coating at a coating amount of 3 mg / tablet. A coating fluid prepared by dissolving 560 g of hydroxypropyl methylcellulose phthalate and 56 g of glyceryl fatty acid ester and dispersing 84 g of a pigment blend (a mixture of iron oxide yellow, titanium oxide, and talc) in 8600 g of 80% ethanol was then sprayed at an inlet air temperature of 60°C to apply a coating at a coating amount of 8 mg / tablet. A coating fluid prepared by dissolving 90 g of Eudragit L, 510 g of ethyl cellulose, and 34 g of cetyl alcohol and dispersing 90 g of talc, 56 g of titanium oxide, and 350 g of magnesium stearate in 13000 g of ethanol was thereafter sprayed at an inlet air temperature of 65°C to apply a coating at a coating amount of 4 mg / tablet.

### Example 2

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then mixed for 50 minutes at 20 rpm using a rotating vessel-type blender (50 L Tumbler Mixer, Tokuju Seisakusho). The mixed granules were compressed (4.8 mmφ, 4.8 mmR) at a compression force of 500 kgf using a rotary tablet press (AP-15, Hata Tekko) to obtain a core.

3000 g of the core was charged into a pan coater (Aqua Coater Model 48, Freund Corporation) and was sprayed at an inlet air temperature of 60°C with a coating fluid prepared by dissolving 186 g of hydroxypropyl cellulose and dispersing 62 g of magnesium stearate in 3600 g of ethanol, to apply a coating at a coating amount of 3 mg / tablet. A coating fluid prepared by dissolving 560 g of hydroxypropyl methylcellulose phthalate and 56 g of glyceryl fatty acid ester and dispersing 84 g of a pigment blend (a mixture of iron oxide yellow, titanium oxide, and talc) in 8600 g of 80% ethanol was then sprayed at an inlet air temperature of 60°C to apply a coating at a coating amount of 8 mg / tablet. A coating fluid prepared by dissolving 90 g of Eudragit L, 510 g of ethyl cellulose, and 34 g of cetyl alcohol and dispersing 90 g of talc, 56 g of titanium oxide, and 350 g of magnesium stearate in 13000 g of ethanol was thereafter sprayed at an inlet air temperature of 65°C to apply a coating at a coating amount of 6 mg / tablet.

### Example 3

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then mixed for 50 minutes at 20 rpm using a rotating vessel-type blender (50 L Tumbler Mixer, Tokuju Seisakusho). The blended granules were compressed (4.8 mmφ, 4.8 mmR) at a compression force of 500 kgf using a rotary tablet press (AP-15, Hata Tekko) to obtain a core.

3000 g of the core was charged into a pan coater (Aqua Coater Model 48, Freund Corporation) and was sprayed at an inlet air temperature of 60°C with a coating fluid prepared by dissolving 186 g of hydroxypropyl cellulose and dispersing 62 g of magnesium stearate in 3600 g of ethanol, to apply a coating at a coating amount of 3 mg / tablet. A coating fluid prepared by dissolving 560 g of hydroxypropyl methylcellulose phthalate and 56 g of glyceryl fatty acid ester and dispersing 84 g of a pigment blend (a mixture of iron oxide yellow, titanium oxide, and talc) in 8600 g of 80% ethanol was then sprayed at an inlet air temperature of 60°C to apply a coating at a coating amount of 8 mg / tablet. A coating fluid prepared by dissolving 90 g of Eudragit L, 510 g of ethyl cellulose, and 34 g of cetyl alcohol and dispersing 90 g of talc, 56 g of titanium oxide, and 350 g of magnesium stearate in 13000 g of ethanol was thereafter sprayed at an inlet air temperature of 65°C to apply a coating at a coating amount of 8 mg / tablet.

### Control Example 1

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then blended for 50 minutes at 20 rpm using a rotating vessel-type blender (50 L Tumbler Mixer, Tokuju Seisakusho). The blended granules were compressed (4.8 mmφ, 4.8 mmR) at a compression force of 500 kgf using a rotary tablet press (AP-15, Hata Tekko) to obtain a core.

3000 g of the core was charged into a pan coater (Aqua Coater Model 48, Freund Corporation) and was sprayed at an inlet air temperature of 60°C with a coating fluid prepared by dissolving 186 g of hydroxypropyl cellulose and dispersing 62 g of magnesium stearate in 3600 g of ethanol, to apply a coating at a coating amount of 3 mg / tablet. A coating fluid prepared by dissolving 560 g of hydroxypropyl methylcellulose phthalate and 56 g of glyceryl fatty acid ester and dispersing 84 g of a pigment blend (a mixture of iron oxide yellow, titanium oxide, and talc) in 8600 g of 80% ethanol was then sprayed at an inlet air temperature of 60°C to apply a coating at a coating amount of 8 mg / tablet.

### Control Example 2

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then blended for 50 minutes at 20 rpm using a rotating vessel-type blender (50 L Tumbler Mixer, Tokuju Seisakusho). The blended granules were compressed (4.8 mmφ, 4.8 mmR) at a compression force of 500 kgf using a rotary tablet press (AP-15, Hata Tekko) to obtain a core.

3000 g of the core was charged into a pan coater (Aqua Coater Model 48, Freund Corporation) and was sprayed at an inlet air temperature of 60°C with a coating fluid prepared by dissolving 186 g of hydroxypropyl cellulose and dispersing 62 g of magnesium stearate in 3600 g of ethanol, to apply a coating at a coating amount of 3 mg / tablet. A coating fluid prepared by dissolving 90 g of Eudragit L, 510 g of ethyl cellulose, and 34 g of cetyl alcohol and dispersing 90 g of talc, 56 g of titanium oxide, and 350 g of magnesium stearate in 13000 g of ethanol was thereafter sprayed at an inlet air temperature of 65°C to apply a coating at a coating amount of 4, 6, 8, 10, 12, or 14 mg / tablet.

### Example 4

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then blended for 50 minutes at 20 rpm using a rotating vessel-type blender (50 L Tumbler Mixer, Tokuju Seisakusho). The blended granules were compressed (4.8 mmφ, 4.8 mmR) at a compression force of 500 kgf using a rotary tablet press (AP-15, Hata Tekko) to obtain a core.

3000 g of the core was charged into a pan coater (Aqua Coater Model 48, Freund Corporation) and was sprayed at an inlet air temperature of 60°C with a coating fluid prepared by dissolving 186 g of hydroxypropyl cellulose and dispersing 62 g of calcium stearate in 3600 g of ethanol, to apply a coating at a coating amount of 3 mg / tablet. A coating fluid prepared by dissolving 560 g of hydroxypropyl methylcellulose phthalate and 56 g of glyceryl fatty acid ester and dispersing 84 g of a pigment blend (a mixture of iron oxide yellow, titanium oxide, and talc) in 8600 g of 80% ethanol was then sprayed at an inlet air temperature of 60°C to apply a coating at a coating amount of 3 mg / tablet. A coating fluid prepared by dissolving 100 g of hydroxypropyl methylcellulose and dispersing 25 g of talc in 1250 g of 60% ethanol was then sprayed at an inlet air temperature of 60°C to apply a coating at a coating amount of 2 mg / tablet. A coating fluid prepared by dissolving 510 g of Eudragit L, 90 g of ethyl cellulose, and 108 g of triethyl citrate and dispersing 90 g of talc, 56 g of titanium oxide, and 350 g of calcium stearate in 13000 g of ethanol was thereafter sprayed at an inlet air temperature of 65°C to apply a coating at a coating amount of 2 mg / tablet.

### Example 5

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then blended for 50 minutes at 20 rpm using a rotating vessel-type blender (50 L Tumbler Mixer, Tokuju Seisakusho). The mixed granules were compressed (4.8 mmφ, 4.8 mmR) at a compression force of 500 kgf using a rotary tablet press (AP-15, Hata Tekko) to obtain a core.

3000 g of the core was added into a pan coater (Aqua Coater Model 48, Freund Corporation) and was sprayed at an inlet air temperature of 60°C with a coating fluid prepared by dissolving 186 g of hydroxypropyl cellulose and dispersing 62 g of calcium stearate in 3600 g of ethanol, to apply a coating at a coating amount of 3 mg / tablet. A coating fluid prepared by dissolving 560 g of hydroxypropyl methylcellulose phthalate and 56 g of glyceryl fatty acid ester and dispersing 84 g of a pigment blend (a mixture of iron oxide yellow, titanium oxide, and talc) in 8600 g of 80% ethanol was then sprayed at an inlet air temperature of 60°C to apply a coating at a coating amount of 3 mg / tablet. A coating fluid prepared by dissolving 100 g of hydroxypropyl methylcellulose and dispersing 25 g of talc in 1250 g of 60% ethanol was then sprayed at an inlet air temperature of 60°C to apply a coating at a coating amount of 2 mg / tablet. A coating fluid prepared by dissolving 510 g of Eudragit L, 90 g of ethyl cellulose, and 108 g of triethyl citrate and dispersing 90 g of talc, 56 g of titanium oxide, and 350 g of calcium stearate in 13000 g of ethanol was thereafter sprayed at an inlet air temperature of 65°C to apply a coating at a coating amount of 4 mg / tablet.

### Example 6

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 hours in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then blended for 50 minutes at 20 rpm using a rotating vessel-type blender (50 L Tumbler Mixer, Tokuju Seisakusho). The mixed granules were compressed (4.8 mmφ, 4.8 mmR) at a compression force of 500 kgf using a rotary tablet press (AP-15, Hata Tekko) to obtain a core.

3000 g of the core was added into a pan coater (Aqua Coater Model 48, Freund Corporation) and was sprayed at an inlet air temperature of 60°C with a coating fluid prepared by dissolving 186 g of hydroxypropyl cellulose and dispersing 62 g of calcium stearate in 3600 g of ethanol, to apply a coating at a coating amount of 3 mg / tablet. A coating fluid prepared by dissolving 560 g of hydroxypropyl methylcellulose phthalate and 56 g of glyceryl fatty acid ester and dispersing 84 g of a pigment blend (a mixture of iron oxide yellow, titanium oxide, and talc) in 8600 g of 80% ethanol was then sprayed at an inlet air temperature of 60°C to apply a coating at a coating amount of 3 mg / tablet. A coating fluid prepared by dissolving 100 g of hydroxypropyl methylcellulose and dispersing 25 g of talc in 1250 g of 60% ethanol was then sprayed at a feed air temperature of 60°C to apply a coating at a coating amount of 2 mg / tablet. A coating fluid prepared by dissolving 510 g of Eudragit L, 90 g of ethyl cellulose, and 108 g of triethyl citrate and dispersing 90 g of talc, 56 g of titanium oxide, and 350 g of calcium stearate in 13000 g of ethanol was thereafter sprayed at an inlet air temperature of 65°C to apply a coating at a coating amount of 6 mg / tablet.

### Control Example 3

2000 g of rabeprazole sodium, 4920 g of mannitol, and 3000 g of crosspovidone were charged into a high shear granulator (100 L Supermixer, Okada Seiko Co., Ltd.) and were mixed for 5 minutes at 490 rpm. Then, while continuing to mix in the high shear granulator at 490 rpm, 4000 g of ethanol containing 100 g of dissolved sodium hydroxide was added into the granulator and granulation was carried out for 5 minutes. The resulting granules were dried for 15 minutes in a tray drier and milled to adjust the size of the granules using a milling device (Power Mill, Dalton Co., Ltd.) and a 1 mmφ mesh screen. The milled granules, 300 g of crosspovidone, and 180 g of sodium stearyl fumarate were then blended for 50 minutes at 20 rpm using a rotating vessel-type blender (50 L Tumbler Mixer, Tokuju Seisakusho). The blended granules were compressed (4.8 mmφ, 4.8 mmR) at a compression force of 500 kgf using a rotary tablet press (AP-15, Hata Tekko) to obtain a core.

3000 g of the core was charged into a pan coater (Aqua Coater Model 48, Freund Corporation) and was sprayed at an inlet air temperature of 60°C with a coating fluid prepared by dissolving 186 g of hydroxypropyl cellulose and dispersing 62 g of calcium stearate in 3600 g of ethanol, to apply a coating at a coating amount of 3 mg / tablet. A coating fluid prepared by dissolving 510 g of Eudragit L, 90 g of ethyl cellulose, and 108 g of triethyl citrate and dispersing 90 g of talc, 56 g of titanium oxide, and 350 g of calcium stearate in 13000 g of ethanol was thereafter sprayed at a feed air temperature of 65°C to apply a coating at a coating amount of 8, 10, 12, or 14 mg / tablet.

### Example 7

A capsule formulation was prepared by sealing 6 tablets of the pulsed-release preparation produced in Example 1 into a #00 gelatin capsule. This capsule formulation was administered orally to beagles. The beagles were fasted for at least 12 hours and received pentagastrin (0.1 mL/animal, s.c.) 30 minutes prior to the administration of the pulsed-release preparation. The excreted material in the feces was checked at the 5th, 8th, and 24th hours after administration of the pulsed-release preparation and the excreted tablet or coating was investigated.

### Control Example 4

A capsule formulation was prepared by sealing the pulsed-release preparation produced in Control Example 2 (amount of controlled-release coating = 12 mg) into a #00 gelatin capsule. This capsule formulation was administered orally to beagles. The beagles were fasted for at least 12 hours and received pentagastrin (0.1 mL/animal, s.c.) 30 minutes prior to the administration of the pulsed-release preparation. The excreted material in the feces was checked at the 5th, 8th, and 24th hours after administration of the pulsed-release preparation and the excreted tablet or coating was investigated.

### Industrial Applicability

By applying an enteric coating to the inside of a controlled-release coating which controls the pulsed-release, the present invention provides a pulsed-release preparation that has a prolonged dissolution lag time. This preparation exhibits the excellent characteristic of being able to extend the dissolution lag time without increasing the coating amount of the controlled-release coating and without increasing the content of the water-insoluble polymer in the controlled-release coating. Moreover, the film strength at the time of pulsed-release is lowered due to the dissolution *in vivo* of the enteric polymer in the intestinal tract, which has a neutral-to-alkaline pH. Accordingly, even for the pulsed-release preparation that has a prolonged dissolution lag time, the excellent features result in a reliable release of the physiologically active substance and the ability to obtain an excellent *in vivo* disintegrability.

### Brief Description of the Drawings

Figure 1 shows the results of dissolution testing on the preparation of Control Example 1, wherein the preparation of Control Example 1 provides dissolution test results for a preparation in which 1) a core containing physiologically active substance and a disintegrant is coated with 2) an enteric coating that covers the core and an inactive intermediate layer is additionally provided between the enteric coating and the core, but which lacks 3) a controlled-release coating containing a water-insoluble polymer and an enteric polymer or water-soluble polymer;
Figure 2 shows the results of dissolution testing on the preparation of Control Example 2, wherein the preparation of Control Example 2 provides release test results for a preparation in which 1) a core containing physiologically active substance and a disintegrant is coated with 3) a controlled-release coating containing a water-insoluble polymer and an enteric polymer or water-soluble polymer with an inactive intermediate layer being provided between the core and the controlled-release coating;
Figure 3 shows the results of dissolution testing on the preparations of Examples 1 to 3 according to the present invention, wherein the preparations of Examples 1 to 3 provide dissolution test results for pulsed-release preparations that comprise 1) a core containing a physiologically active substance and a disintegrant; 2) an enteric coating that covers the core, and 3) a controlled-release coating that covers the enteric coating applied on the core and that contains a water-insoluble polymer and an enteric polymer or water-soluble polymer, and that additionally comprise an inactive intermediate coating provided between the core and the enteric coating;
Figure 4 shows the results of dissolution testing on the preparation of Control Example 3, wherein the preparation of Control Example 3 provides dissolution test results for a preparation in which 1) a core containing physiologically active substance and a disintegrant is coated with 3) a controlled-release coating containing a water-insoluble polymer and an enteric polymer or water-soluble polymer with an inactive intermediate layer being provided between the core and the controlled-release coating, but which lacks an enteric coating that covers the core; and
Figure 5 shows the results of dissolution testing on preparations (Examples 4 to 6) according to the present invention, wherein the preparations of Examples 4 to 6 provide dissolution test results for the pulsed-release preparations that comprise 1) a core containing a physiologically active substance and a disintegrant; 2) an enteric coating that covers the core; and 3) a controlled-release coating that covers the enteric coating applied on the core and that contains a water-insoluble polymer and an enteric polymer or water-soluble polymer, and that additionally comprise an inactive intermediate coating provided between the enteric coating and the controlled-release coating and also an inactive intermediate coating provided between the core and the enteric coating.

## Claims

1. A pulsed-release preparation comprising:
1) a core comprising a physiologically active substance and a disintegrant;
2) an enteric coating which covers the core, and which comprises a first enteric polymer; and
3) a controlled-release coating which covers the enteric coating, and which comprises a water-insoluble polymer and a second enteric polymer or a water-soluble polymer.

2. The pulsed-release preparation according to Claim 1, further comprising a first inactive intermediate coating provided between the core and the enteric coating.

3. The pulsed-release preparation according to Claim 1 or 2, further comprising a second inactive intermediate coating provided between the enteric coating and the controlled-release coating.

4. The pulsed-release preparation according to any one of Claims 1 to 3, wherein the disintegrant is at least one selected from the group consisting of crosspovidone, low-substituted hydroxypropyl cellulose, crosscarmellose sodium, and carmellose calcium.

5. The pulsed-release preparation according to any one of Claims 1 to 4, wherein the water-insoluble polymer is at least one selected from the group consisting of ethyl cellulose, aminoalkyl methacrylate copolymer RS (Eudragit RS), and shellac.

6. The pulsed-release preparation according to any one of Claims 1 to 5, wherein the first and second enteric polymers are at least one selected from the group consisting of hydroxypropyl methylcellulose phthalate, hydroxypropyl methylcellulose acetate succinate, methacrylic acid-methyl methacrylate copolymer (Eudragit L, Eudragit S), and methacrylic acid-ethyl acrylate copolymer (Eudragit LD).

7. The pulsed-release preparation according to any one of Claims 1 to 6, wherein the water-soluble polymer is at least one selected from the group consisting of hydroxypropyl cellulose, methyl cellulose, sodium carboxymethyl cellulose, hydroxypropyl methylcellulose, and polyvinylpyrrolidone.

8. The pulsed-release preparation according to any one of Claims 1 to 7, wherein the physiologically active substance is a physiologically active substance that is unstable to acid.

9. The pulsed-release preparation according to Claim 8, wherein the physiologically active substance that is unstable to acid is a benzimidazole-type compound or a pharmacologically acceptable salt thereof.

10. The pulsed-release preparation according to Claim 9, wherein the benzimidazole-type compound or pharmacologically acceptable salt thereof is rabeprazole, omeprazole, pantoprazole, lansoprazole, esomeprazole, or a pharmacologically acceptable salt thereof.

11. The pulsed-release preparation according to Claim 10, wherein the rabeprazole or pharmacologically acceptable salt thereof is rabeprazole sodium.

12. The pulsed-release preparation according to any one of Claims 1 to 11, wherein the core further comprises an alkaline additive.

13. The pulses-release preparation according to any one of Claims 1 to 12, wherein the pulsed-release preparation is a tablet, a granule, or a fine granule.

14. A capsule formulation comprising:
the pulsed-release preparation according to any one of Claims 1 to 13; and
an enteric preparation comprising an enteric coating provided on a core that comprises a physiologically active substance that is unstable to acid.

15. A process for producing a pulsed-release preparation, comprising the steps of:
forming an enteric coating which covers a core, and which comprises a first enteric polymer, the core comprising a physiologically active substance and a disintegrant; and
forming a controlled-release coating which covers the enteric coating, and which comprises a water-insoluble polymer and a second enteric polymer or a water-soluble polymer.
